# EUROPEAN PATENT SPECIFICATION

(11) **EP 0 256 654 B1**
(45) Date of publication and mention of the grant of the patent: **18.09.1996**
(21) Application number: 87306006.5
(22) Date of filing: 07.07.1987
(51) Int. Cl.: C12N 15/13, C12P 21/00, C12P 21/08, A61K 39/395

(54) **Chimeric murine/human immunoglobulin specific for tumour-associated 17-1A Antigen**
Chimärisches Murine-Mensch-Immunoglobulin, spezifisch für tumorassoziertes 17-1A Antigen
Immunoglobuline chimère murine-humaine, spécifique pour l' antigène 17-1A associés aux tumeurs

(30) Priority: 07.07.1986 US 882492; 01.07.1987 US 69414
(43) Date of publication of application: 24.02.1988
(73) Proprietor: CENTOCOR, INC., Malvern, PA 19355 (US)
(72) Inventor: Schoemaker, Hubert J.P., Devon, PA 19333 (US); Sun, Lee K., Media, PA 19063 (US)
(74) Representative: Holdcroft, James Gerald, Dr.

(56) References cited:
- EP-A- 0 171 496
- EP-A- 0 184 187
- WO-A-86/01533
- WO-A-87/02671
- SCIENCE, vol. 229, 20 September 1985, Washington, DC (US); S.L. MORRISON, pp. 1202-1207
- THE LANCET, no. 8481, 15 March 1986, London (GB); R.W. BALDWIN et al., pp. 603-605
- NATURE, vol. 314, no. 6010, April 1985, London (GB); S. TAKEDA et al., pp. 452-454
- PROCEEDINGS OF THE NATL. ACADEMY OF SCIENCE USA, vol. 81, November 1984, Washington, DC (US); S.L. MORRISON et al., pp. 6851-6855
- PROCEEDINGS OF THE NATL. ACADEMY OF SCIENCE USA, vol. 84, January 1987, Washington, DC (US); L.K. SUN et al., pp. 214-218
- JOURNAL OF IMMUNOLOGICAL METHODS, vol. 85, 1985; D. HERLYN et al., pp. 27-38
- JOURNAL OF EXPERIMENTAL MEDICINE, vol. 163, 1986; R.J. BENJAMIN et al., pp. 1539-1552
- JOURNAL OF IMMUNOLOGY, vol. 135, no. 2, 1985; D.L. SHAWLER et al., pp. 1530-1535
- ANTIBODY, IMMUNOCONJUGATES & RADIOPHARMACEUTICALS, vol. 5, no. 1, 1992; R.F. MEREDITH et al., pp. 75-80

## Description

### Background

Monoclonal antibodies have been developed against antigens associated with tumor for use in therapy of cancer. One example is the murine monoclonal antibody 17-1A which is directed against a surface antigen of colorectal carcinoma cells and other tumor cells. The antibody has been used for immunotherapy of colon cancer, and in several instances, the treatment has resulted in partial or complete regression of colorectal carcinoma.

Most monoclonal antibodies that have been developed against tumor associated antigens are murine antibodies. The principle reason is that techniques for production of monoclonal antibodies in this species are well developed. Human monoclonal antibodies are difficult to produce. The standard technique used to make murine hybridomas-immunizing with antigen and harvesting spleen cells for fusion-obviously cannot be used to produce human monoclonal antibodies. Human lymphocytes which produce antibodies against a particular antigen can be selected from persons naturally exposed to the antigen but hybridomas formed with human cells are often unstable and cease producing antibodies.

The success of the murine system for production of monoclonal antibodies provides a means to produce monoclonal antibodies against many types of cancer antigens. Murine antibodies, however, have several drawbacks which may severely limit the use in human cancer therapy. As foreign proteins, murine antibodies often elicit counteracting immune reactions which may reduce or destroy their therapeutic effectiveness. In addition, murine antibodies can evoke allergic reactions in patients. Unfortunately, the need for readministration in therapy increases the likelihood of these immune reactions in patients.

A method which has been suggested to circumvent these problems is to produce antibodies consisting of mouse variable regions joined to human constant regions. See, e.g., Morrison, S. L. et al. (1984) "Chimeric Human Antibody Molecules: Mouse Antigen-binding Domains with Human Constant Region Domains" Proc. Natl. Acad. Sci. USA 81:6851; Neuberger, M. S. and Rabbits, T. H. "Production of Chimeric Antibodies" PCT Application No. PCT/GB85 00392. These chimeric antibodies would have the antigen binding specificity of the murine antibody but because they have human constant regions they would be, for the most part, human antibodies. Because the constant region is largely responsible for immune response against antibody, chimeric murine/human antibodies with constant regions are of human origin should be less likely to evoke an anti-murine immune response in humans. Further, the human constant region may provide for an antibody with better effector function in humans.

The joining of a human constant region to a murine variable region might alter the conformation of the variable region in a manner which reduces or destroys the binding capability of the resulting chimera. Chimeric murine/murine and murine/human antibodies having variable region for haptens such as azophenylarsonate and trinitrophenyl have been produced and these antibodies have been shown to retain the original hapten-binding specificity of the murine antibody from which the variable regions are derived. See, for example, Sharon, J. et al. (1984) "Expression of a V_{H}C_{K} Chimeric Protein in Mouse Myeloma Cells" Nature 309:364-366; Boulianne, G. L. et al., (1984) "Production of Functional Chimeric Mouse/ Human Antibody", Nature, 312: 604-608; and Neuberger, M. S. et al., "A Hapten Specific Chimeric IgE Antibody with Human Physiological Effector Function" (1985) Nature 314: 268-270. However, because hapten molecules are small, conformational changes which may be induced in the murine variable region by the attachment of a human constant region may not significantly alter binding to such molecules. When the cognate antigen is a large molecule such as a tumor or proliferation associated cell surface antigen, conformational changes in the region could substantially influence binding capability.

EP-A-0171496 discloses chimeric monoclonal antibodies and genes encoding chimeric antibodies specific for a tumour associated antigen.

Herlyn et al, P.N.A.S. USA 76(3) (1979) pp 1483-1442 discloses the 17-1A monoclonal antibody and producer cell-line.

Herlyn et al (1985) J. Immunological Methods, 85; pages 27-38 discloses anti-idiotypic responses to monoclonal anti-colon carcinoma antibody 17-1A.

Benjamin et al (1986) J. Exp. Med. 163, pp 1539-1552 disclose anti-idiotypic responses to chimeric antibodies reactive with cell-surface antigens.

Shawler et al (1985) Journal of Immunology 135 (2), pages 1530-1535 disclose anti-idiotypic responses to human monoclonal antibodies following multiple infusions.

### Disclosure of the Invention

This invention pertains to murine/human chimeric immuno-globulins as defined in claim 1.

The chimeric immunoglobulins comprise:
a) at least one chimeric heavy chain comprising an antigen-binding region derived from the heavy chain of a murine antibody specific for the antigen defined by the 17-1A murine monoclonal antibody linked to a human heavy chain constant region; and
b) at least one chimeric light chain comprising an antigen-binding region derived from a light chain of the murine antibody linked to a human light chain constant region, the chimeric immunoglobulin retaining specificity for the antigen defined by the 17-1A murine monoclonal antibody.

The immunoglobulins can be monovalent dimers divalent tetramers or polyvalent aggregates. These chimeric mouse/human immunoglobulins retain the original binding specificity for the antigen.

The constant regions of the chimeric immunoglobulin are derived from human immunoglobulins. The constant region of the heavy chain can be selected from any isotype class or subclass and the constant region of the light chain can be of the kappa or lambda class.

The chimeric antibodies are produced by cloning DNA segments encoding the heavy and light chain variable regions of a murine antibody specific for a tumor-associated antigen and joining these DNA Segments to DNA segments encoding human heavy and light chain constant regions to produce chimeric immunoglobulin encoding genes. The fused gene constructs coding for the light and heavy chain are assembled in or inserted into expression vectors. The genes are cotransfected into a lymphoid recipient cell (e.g. a myeloma cell) where the immunoglobulin proteins can be synthesized, assembled and secreted.

The chimeric antibodies of this invention are useful for therapy and diagnosis of cancer. The chimeric antibodies may be used for tumor therapy alone or with several other immunoglobulins in mixtures or "cocktails" of antibodies specific for different antigens exhibited by a heterogenous tumor. In another mode of therapy, the chimeric immunoglobulins can be conjugated, either chemically or by genetic engineering techniques, to cancer therapeutic agents to form so-called "smart" drugs specifically directed to cancer cells. For diagnostic use, the chimeric immunoglobulins can provide agents for tumor imaging. For example, the immuno-globulins can be made with truncated heavy chains to provide "antibody fragments" and labeled either directly or through a label chelator. The chimeric immunoglobulins can be designed to contain a "built-in" label chelating protein or protein domain.

The use of chimeric murine/human antibodies in vivo offers several advantages over the use of murine monoclonal antibody. First, these antibodies may be less immunogenic in eliciting human antimouse reactions in patients. Second, a human constant region of choice can be linked to a mouse variable region gene to produce antibodies with the desired biological effector functions. Finally, it is possible to express hybrid genes constructed by joining Ig DNA and non-Ig DNA sequences. As mentioned above, these fusion proteins constitute antibody based delivery systems for selective destruction or imaging of tumor cells.

### Brief Description of the Drawings

Figure 1 shows the nucleotide sequences of the 17-1A. functional light chain (A) and heavy chain (B) genes and the predicted amino acid sequences.

Figure 2 shows the Southern blot analysis of rearranged 17-1A V_{H} genes containing J_{H} sequences.

Figure 3 shows the structure of the chimeric heavy and light chain vectors A, pSV184ΔHneo17-1AV_{K}-hC_{K}; B, pSV2ΔHgpt17-1AV_{H}-hC_{γ}3.

Figure 4 shows SDS/PAGE of ³⁵ S-methionine labeled chimeric immunoglobulin proteins of transfected Sp2/0 cell line SG3/5 and SG3/7.

Figure 5 shows the inhibition of 17-1A binding to SW1116 cells by the chimeric immunoglobulin SG3/5.

Figure 6 shows the structure of the chimeric heavy chain vector pSV2ΔHgpt17-1AV_{H}-hC_{µ}.

### Detailed Description of the Invention

The chimeric immunoglobulins of this invention are comprised of individual chimeric heavy and light, immunoglobulin chains. The chimeric heavy chain is a contiguous polypeptide having a murine heavy chain variable region and a human heavy chain constant region. The chimeric light chain is a contiguous polypeptide having a murine light chain variable region and human light chain constant region.

The immunoglobulins can be monovalent, divalent or polyvalent. Monovalent immunoglobulins are dimers (HL) formed of a chimeric heavy chain associated (through disulfide bridges) with a chimeric light chain. Divalent immunoglobulins are tetramers (H₂L₂) formed of two associated dimers. Polyvalent immunoglobulins can be produced, for example, by employing heavy chain constant region which aggregate (e.g., mu type constant regions).

The heavy chain constant region can be selected from any of the five isotypes alpha, delta, epsilon, gamma or mu. Further, heavy chains of various subclasses (such as the IgG subclasses) can be used. The different classes and subclasses of heavy chains are responsible for different effector functions and thus, by choosing the desired heavy chain constant region, chimeric antibodies with desired effector function can be produced. Preferred constant regions are gamma 1 (IgG1) and gamma 3 (IgG3). The light chain constant region can be the kappa or lambda chain.

In general, the chimeric antibodies are produced by preparing, for each of the light and heavy chains components of the chimeric immunoglobulin, a fused gene comprising a first DNA segment which encodes at least the functional portion of the murine variable region linked to a second DNA segment encoding at least a part of a human constant region. Each fused gene is assembled in or inserted into an expression vector. Recipient cells capable of expressing the gene products are then transfected with the genes. The transfected recipient cells are cultured and the expressed immunoglobulins or immunoglobulin chains are recovered.

Genes encoding the variable region of murine Ig light and heavy chains can be obtained from lymphoid cells which produce the antibodies specific for the desired tumor antigen. For example, the hybridoma cell lines which produce antibody against the antigens 17-1A, OC125 or any of the other antigens provide a source of immunoglobulin variable region genes against certain tumor associated antigens. Other rodent cell lines are available. Cell lines can be produced by challenging a rodent with a tumor cell or a tumor antigen-containing cell component or fraction, forming fused hybrid cells between antibody producing cells and a myeloma, cloning the hybrid and selecting clones which produce antibody against tumor-associated antigen. See U.S. Patent No. 4,172,124 Koprowski et al.

Constant regions can be obtained from human antibody producing cells by standard cloning techniques. Alternatively, because genes representing the two classes of light chains and the five classes of heavy chains have been cloned, constant regions of human origin are readily available from these clones.

Preferably, the fused genes encoding the light and heavy chimeric chains are assembled in two different expression vectors which can be used to cotransform a recipient cell. Each vector contains two selectable genes-one for selection in a bacterial system and one for selection in a eukaryotic system- each vector having a different pair of genes. These vectors allow production and amplification of the fused genes in bacterial systems and subsequent cotransfection of eukaryotic cells and selection of the cotransfected cells. Examples of selectable gene for the bacterial system are the genes which confer ampicillin and the gene which confers chloramphenicol resistance. Two selectable genes for selection of eukaryotic transfectants are preferred: (i) the xanthine-guanine phosphoribosyltransferase gene (gpt), and (ii) the phosphotransferase gene from Tn5 (designated neo). Selection with gpt is based on the ability of the enzyme encoded by this gene to use xanthine as a substrate for purine nucleotide synthesis; the analogous endogenous enzyme cannot. In a medium containing xanthine and mycophenolic acid which blocks the conversion of inosine monophosphate to xanthine monophosphate, only cells expressing the gpt gene can survive. The product of the neo blocks the inhibition of protein synthesis in eukaryotic cells caused by the antibiotic G418 and other antibiotics of its class. The two selection procedures can be used simultaneously or sequentially to select for the expression of immunoglobulin chain genes introduced on two different DNA vectors into a eukaryotic cell.

The preferred recipient cell line is a myeloma cell. Myeloma cells can synthesize, assemble and secrete immunoglobulins encoded by transfected Ig genes. Further, they possess the mechanism for glycosylation of the immunoglobulin. A particularly preferred recipient cell is the Ig-nonproducing myeloma cell Sp2/0. The cell produces only immunoglobulin encoded by the transfected immunoglobulin genes. Myeloma cells can be grown in culture or in the peritoneum of mice where secreted immunoglobulin can be obtained from ascites fluid. Other lymphoid cells such as B lymphocytes or hybridoma cells can serve as suitable recipient cells.

Several methods exist for transfecting lymphoid cell with vectors containing immunoglobulin encoding genes. A preferred way of introducing DNA into lymphoid cells is by protoplast fusion. In this method, lysozyme is used to strip cell walls from bacteria harboring the recombinant plasmid containing the chimeric Ig gene. The resulting spheroplasts are fused with myeloma cells with polyethylene glycol. After protoplast fusion, the transfectants are selected and isolated. Another technique which can be used to introduce DNA into many cell types is calcium phosphate precipitation.

The chimeric immunoglobulin genes can be expressed in nonlymphoid cells such as bacteria or yeast. When expressed in bacteria, the immunoglobulin heavy chains and light chains become part of inclusion bodies. Thus, the chains must be isolated and purified and then assembled into functional immunoglobulin molecules.

A functional chimeric antibody specific for the 17-1A antigen associated with colorectal carcinoma and some other forms of cancer was prepared as follows.

Genomic DNA segments encoding the heavy and light chain V regions of 17-1A (IgG2a, kappa light chain) were cloned and then joined to DNA segments encoding human γ3 constant (C _{γ3}) and kappa constant (C_{K}) regions. The recombinant genes were then transfected into mouse myeloma cells where the Ig proteins were synthesized, assembled and secreted. To obtain probes for the V regions, a cDNA library for 17-1A was constructed. RNA was extracted and polyA⁺ RNA prepared by oligodT cellulose chromatography. Double-stranded cDNA was synthesized with polyA⁺ RNA as template using AMV reverse transcriptase and E. coli DNA polymerase I. Double-stranded cDNA was treated with S₁ nuclease and elongated with deoxycytidine residues, and annealed with dG-tailed pUC8 previously cut with PstI. The recombinant plasmids were transformed into E. coli DH1, and colonies screened using a mouse C_{K} probe and a mouse C _{γ2a} probe to isolate the light and heavy chain clones, respectively. These clones were used as a source of V region probes.

To isolate the genomic DNA fragment containing the functionally rearranged kappa light chain gene, a genomic library was constructed from Sau3A partially digested DNA of 17-1A using EMBL3A phage arms. A total of 200,000 recombinant plaques were screened with a mouse C_{K} probe, three positive cones were obtained. One of these clones, λ_{K}4, was shown to contain both the V and C regions of the functionally rearranged kappa light chain gene of 17-1A by restriction mapping and Southern analysis using the 17-1A _{K} cDNA as a probe. When the same procedure was repeated using a mouse C _{γ2a} probe and the heavy chain V(V_{H}) probe derived from the cDNA clone, no positive clones were found to contain both the V and C regions of the heavy chain gene.

An alternative approach was taken to clone the V_{H} gene. Fragments containing the rearranged heavy chain V genes of 17-1A were identified by Southern analysis as additional bands when compared with the pattern of the fusion partner, NS-1. When a DNA sample of 17-1A cells was digested with restriction enzyme EcoRI, two rearranged fragments of 7.4kb and 3.8kb were noted using a mouse heavy chain J region (J_{H}) probe containing J3 and J4 sequences. DNA fragments of ∼7kb and ∼4kb isolated by preparative agarose gel electrophoresis were ligated with λgtWES and λgt11 phage arms, respectively. The recombinant plaques of these two partial libraries were screened with a mouse J_{H} probe. Both 7.4kb and 3.8kb EcoRI fragments were cloned. The 7.4kb fragment was shown to contain the functionally rearranged V_{H} gene by restriction mapping and Southern analysis using the 17-1A heavy chain cDNA as a probe.

The V_{K} gene was isolated in a 4kb HindIII fragment from the genomic clone λ_{K}4 and joined to the human C_{K} sequences in the expression vector pACYCSVneo. The direction of the K gene transcription is opposite that of the neo gene. The 7.4kb EcoRI fragment containing the V_{H} gene was joined to the human C _{γ3} gene in the expression vector pSV2gpt with the direction of transcription opposite that of Ecogpt. Since the pACYCSVneo plasmid confers chloramphenicol resistance and the pSV29gpt plasmid confers ampicillin resistance, it is possible to transform E. coli with both plasmids and select cells expressing dual drug resistance phenotypes. To transfer the Ig genes into myeloma cells, E. coli harboring both plasmids were converted to protoplasts and fused with a nonproducing mouse myeloma line, Sp2/0. After protoplast fusion, the transfected cells were selected for neo gene activity by treatment with G418. To examine heavy and light chain protein synthesis, transfected cells were labeled with ³⁵S-methionine, both cytoplasmic extract and secreted material were immunoprecipitated with Sepharose-bound goat anti-human K antibody. The precipitated material was analyzed on SDS/polyacrylamide gels containing phosphate buffer under nonreducing conditions. From 4x10⁶ cells, seven stable transformants were established and analyzed. Two of these clones produced both heavy and light chain proteins, two produced only light chain proteins, and three did not produce any detectable amounts of Ig proteins. The clone SG3/5, one of the two transformants that produced both heavy and light chain proteins produced a mixture of H₂L₂, H₂L and HL molecules. The major components secreted into the culture medium were the tetrameric molecules, H₂L₂. The chimeric H₂L₂ immunogloblin displayed the same antigen binding properties as the urine 17-1A antibody.

Chimeric murine/human antibodies specific for cancer-associated antigens can be used for therapy or diagnosis of cancer. For therapy, the antibodies can be used in a number of different ways. Certain antibodies are capable of specifically binding to and killing tumor cells by themselves. Chimeric antibodies having the variable regions of such antibodies can be administered in anti-tumor amounts to patients to treat tumor. The chimeric murine/human 17-1A antibody can be administered to patients bearing gastrointestinal tumors with which the 17-1A antigen is associated.

Cancer cells are heterogenous and consequently, a single monospecific chimeric antibody may not be able to recognize all cells of a tumor. Thus, it may be desirable to administer several chimeric antibodies of different antigenic specificity in combination.

The chimeric antibodies can be used in conjunction with other anti-tumor agents. For this purpose, the chimeric murine/human antibodies can be conjugated to certain therapeutic agents or cytotoxic agents or to functional domains of such proteins. In this capacity, the immunoglobulins would act as delivery vehicles specifically targeted for cancer cells. Such conjugation can be accomplished by chemically coupling the therapeutic agent to the antibody. In cases where the therapeutic agent is proteinaceous, the conjugation can be accomplished by linking an agent-encoding DNA sequence to the chimeric gene encoding an antibody chain (preferably a heavy chain) so that the antibody chain and agent are expressed as a single contiguous protein in which the agent is linked by a peptide bond to the immunoglobulin. In this way, chimeric immunoglobulins containing immunoglobulin and nonimmunoglobulin portions can be formed where the nonimmunoglobulin portion can be a selected therapeutic agent such as interferon or selected cytoxic agents such as tumor necrosis factor.

The chimeric antibodies are also useful in vivo diagnostic techniques such as immunoscintigraphy. For this purpose, antibody fragments are generally preferred. Thus, a chimeric heavy chain gene can be designed in truncated form to produce chimeric Fab immunoglobulin fragment or a chimeric F(ab')₂-like molecule for tumor imaging. These molecules can be labeled directly or through a coupled chelating agent with radioisotopes such as ¹³¹Iodine, ¹²⁵ Iodine, ^{99m}Technetium or ¹¹¹Indium to produce radioimmunoscintigraphic agents for tumor imaging. Alternatively, a radiometal binding (chelating) domain can be engineered into the chimeric antibody for labeling a site. Thus, a chimeric immunoglobulin can be designed as a contiguous protein which has a murine variable region, a human constant region (preferably truncated), and a metal binding domain derived from metal binding protein such as metallothionine.

The invention is illustrated further by the following detailed examples describing the production of the chimeric antibody with 17-1A derived variable regions and human constant regions.

### EXAMPLES

### Example 1. Production of chimeric 17-1A IgG3.

### A. Material and Methods

### cDNA library construction.

Cytoplasmic RNA was extracted from 17-1A cells and polyA⁺ RNA was prepared by oligo-dT cellulose chromatography. Double-stranded cDNA was synthesized with polyA⁺ RNA as a template using AMV reverse transcriptase and E. coli DNA polymerase I. Double-stranded cDNA was treated with S1 nuclease and elongated with deoxycytidine residues, and annealed with dG-tailed pUC8 previously cut with PstI. Curtis, P.J., (1983) J. Biol. Chem. 258:4459. The recombinant plasmids were transformed into E. coli DH1, and colonies screened according to the general method described by Maniatis, T. et al. (1982) Molecular Cloning. A Laboratory Manual.

### Genomic library construction.

Genomic library was constructed in lambda phage vector EMBL3A. High molecular weight DNA was partially digested with restriction endonuclease Sau3A and size-fractionated on a 10-40% sucrose density gradient. DNA fragments of 18-23kb were ligated with EMBL3A lambda arms and packaged according to Hohn and Murray (1977) Proc. Natl. Acad. Sci USA 74: 3259. The genomic library was screened at a density of 10,000 recombinant plaques per 150mm diameter petri dish. Plaque hybridizations were carried out in 5x SSC at 65° for 18h. Final washes were in 1.0x or 0.5x SSC at 65°.

### Partial genomic library construction.

High molecular weight genomic DNA was digested to completion with restriction endonuclease and fract onated on a 0.8% agarose gel. DNA fragments of the appropriate size was isolated and ligated with lambda phage arms. The ligated DNA were packaged and recombinant plaques were screened as described above.

### DNA analysis.

Genomic DNA was digested with restriction endonucleases fractionated by electrophoresis through a 0.7% agarose gel, and blotted to nitrocellulose. See Maniatis et al. supra. Hybridizations were in 5x SSC and 50% formamide at 37° for 48h. Final washes were 0.5x SSC at 65°.

### DNA probes.

The mouse γ 2a probe is a 4.9 kb EcoRI DNA fragment containing the γ 2a constant region gene. The mouse Cₖ probe is a 6 kb BgIII genomic DNA fragment containing the mouse k light chain constant region sequences. The mouse heavy chain J (J_{H}) probe is a 2 kb BamHI/EcoRI fragment containing both J3 and J4 segments. ³²P-labeled probes were prepared by using calf thymus primers. Summers, J. (1975) J. Virol 15:946. Free nucleotides were removed by centrifugation through a Sephadex G-75 mini-column.

### Gene transfer by protoplast fusion.

The construction of pSV184neo- and pSV2gpt-derived by plasmids carrying the chimeric light and heavy chain genes is described below. E. coli HB101 harboring both plasmids were grown in the presence of ampicillin and chloramphenicol. The plasmid copy number was amplified with spectinomycin at l00ug/ml. Protoplasts were prepared and fused to mouse myeloma Sp2/0 cells according to Oi, et. al., (1983) Proc. Natl. Acad. Sci. USA 80:825. Transformants were selected in Dulbecco's modified Eagle's medium containing antibiotic G418 at 0.8 mg/ml, supplemented with 15% fetal calf serum. The SG3/5 cell line was maintained in the above medium plus xanthine at 50 µg/ml, hypoxanthine at 4 µg/ml and mycophenolic acid at 0.8 µg/ml.

### Analysis of Ig by biosynthetic labeling and immunoprecipitation.

Cells were labeled for 3h in methionine-free RPMI 1640 medium containing ³⁵S-methionine at 25µCi/ml. Affinity-purified goat anti-human K light chain antibody (Southern Biotechnology Associated, Inc., Birmingham, AL) and goat anti-human IgG Fc antibody (Jackson Immunoresearch Laboratories, Inc., Avondale, PA) were used for immunoprecipitations. Morrison, S.L. (1979) J. Immunol. 123:793. Both cytoplasmic and secreted antibodies were analyzed on a 5% SDS/polyacrylamide gel in phosphate buffer under nonreducing conditions. Matsuuchi, L. et al. (1981) Biochem. 20:4827. Gel was treated with an autoradiography enhancer, EN3HANCE (NEN Products, Boston, MA) before drying down onto 3mm filter paper.

### Quantitation of antibody production.

Tissue culture supernatant was analyzed for IgG protein content by particle concentration fluorescence immunoassay (Jolley, M.E. et al., (1984) J. Immunol. Meth. 67:21 using standard curves generated with purified IgG. Concentration of MAb 17-1A was determined using goat anti-mouse Fab antibody-coated polystyrene beads and fluorescein conjugated antimouse Fab antibody. Chimeric antibody with human constant regions was determined using goat antihuman IgG Fc antibody-coated polystyrene beads and fluorescein conjugated goat anti-human TgG Fc antibody. The assay was carried out with an automated instrument (Pandex Laboratories, Inc., Mundelein, IL).

### Radioiodination of MAb 17-1A.

MAb 17-1A was purified from ascites fluid by using protein A-Sepharose chromatography. Bound IgG was eluted with a pH 3.5 citrate buffer. Purified MAb 17-1A was labeled with 1.4 mM choramine T using 1 mCi Na¹²⁵I. After 3 min, the reaction was quenched with excess ascorbic acid. Free iodide was removed with a prepacked PD-10 column (Pharmacia). Specific activity was typically 10,000cpm/ng of protein.

### Binding inhibition assay.

Tissue culture supernatant was concentrated with a Diaflo YM100 ultrafiltration membrane (Amicon, Danvers, MA). Monolayer colorectal carcinoma cells were trypsinized and washed in phosphatebuffer saline (PBS). Cells (5x10⁵) were incubated with radioiodinated 17-1A and culture supernatant containing the competing antibody in a final volume of 100 ul. Incubation was done at room temperature for 2 hr in a shaker at 140rpm. The cells were washed with PBS and cell-bound radioactivity counted in a gamma counter.

### B. Results

### Isolation and sequencing of the light and heavy chain cDNA of 17-1A.

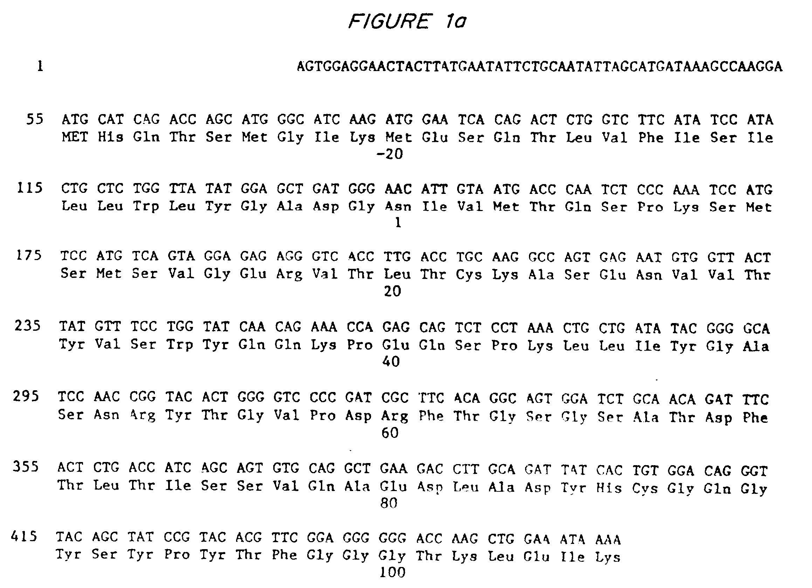

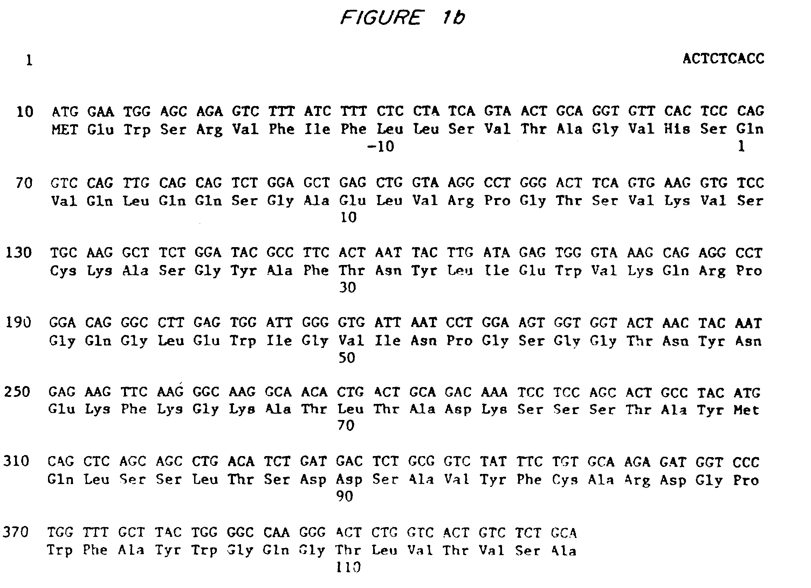

A CDNA library was prepared for the 17-1A cells using plasmid vector pUC8. Recombinant colonies were screened with mouse C_{K} probe and the mouse Cγ2a probe to isolate the light and heavy chain clones, respectively. A total of 7,500 colonies were screened, approximately 250 of which contained CK sequences and 150 contained Cγ2a sequences. Plasmid DNA prepared from some of these positive colonies was digested with PstI to compare the size of the cDNA inserts. The light chain clone, pMK-9, and the heavy chain clone, pM γ2a-1, were chosen for nucleotide sequencing. The nucleotide sequences for the 5' region containing the leader peptide and the variable region, and the predicted amino acid sequences are shown in Figure 1A for pMK-9 and in Figure 1B for pMγ2a-1. (Amino acid residues are numbered, and the negative numbers refer to amino acids in the leader peptide). A variable region-specific (V_{L}) probe was derived from pMK-9 as a BamHI/PvuI fragment which contained the first 320 nucleotides of the light chain gene (Figure 1A). The heavy chain V region probes contained two PstI fragments: the 228bp V_{H}1 and the 132bp V_{H}2 probes corresponding to nucleotide 52 to 280, and 281 to 412, respectively (Figure 1B). These V region probes were used in subsequent experiments to characterize the genomic DNA fragments containing functionally rearranged genes.

### Cloning of the genomic DNA fragment containing the functionally rearranged light and heavy chain genes of 17-1A.

A genomic DNA library was prepared for the 17-1A cells. Approximately 200,000 lambda phage recombinants were screened with the mouse C_{K} probe. Three positive clones were obtained. One of the clones, λK4, was shown to contain the variable region sequences by restriction mapping and Southern analysis using the V_{L} probe derived from the cDNA clone, pMK-9. A 4.2 kb HindIII genomic DNA fragment containing 1.5 kb of the 5' flanking region and the sequences encoding the leader peptide and the V gene was subcloned into pUC 18, and designated as pV_{K}4.2H. This subclone was used in the subsequent construction of the mouse/human chimeric light chain gene.

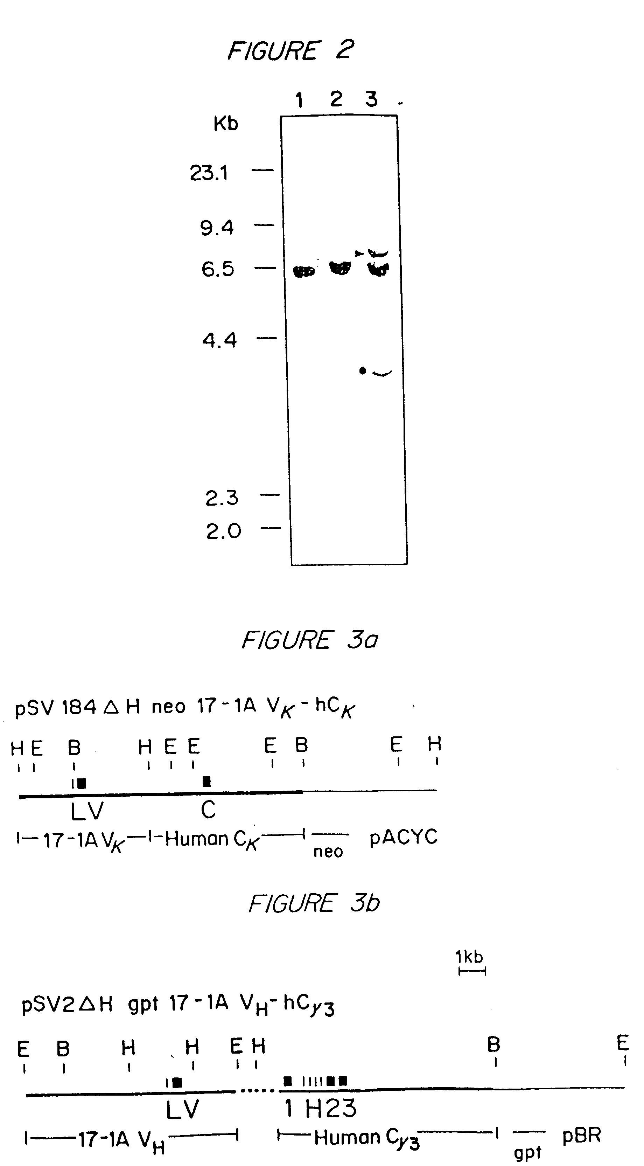

When the 17-1A genomic library was screened with the mouse C_{γ} 2a probe, two positive clones were obtained, but neither of these contained the heavy chain variable (V_{H}) gene of the cDNA clone, pM γ2a-1. An alternative approach was taken to clone the V_{H} gene. During a gene rearrangement that is required for Ig gene expression, the V gene is always associated with the appropriate J segment. The DNA probe J_{H} can therefor be used to detect the rearranged V_{H} genes. Figure 2 shows the Southern analysis of rearranged fragments containing J_{H} sequences. (Ten µg of genomic DNA was digested with EcoRI, fractionated on 0.7% agarose, transferred to nitrocellulose, and the filter hybridized with the mouse heavy chain J_{H} probe. Lane 1, mouse liver DNA; lane 2, P3, a mouse plasmacytoma cell line; lane 3, 17-1A, a hybridoma cell line derived by using P3 as a fusion partner. The arrowhead indicates the rearranged fragment containing the functional V_{H} gene of 17-1A. The solid circle indicates additional rearrangement in the heavy chain locus).

DNA of 17-1A cells showed two rearranged EcoRI fragments at 7.4 kb and 3.8 kb in addition to the band characteristic of the fusion partner, P3. The P3 cells had two rearranged V_{H} genes which comigrated at 6 kb. One of the two bands of 17-1A represented the functional rearrangement, while the other was the product of an aberrant gene rearrangement in the heavy chain locus. Using the mouse J_{H} probe, both genes were cloned from phage genomic libraries constructed with enriched DNA fragments of appropriate sizes. Clone λV_{H} 7.4E was isolated from a λgtWES library and the EcoRI insert was found to comigrate with the 7.4 kb rearranged fragment, as indicated with an arrowhead in Figure 2. Clone λV_{H}3.8E was isolated from a λgt11 library and its EcoRi insert comigrated with the 3.6 kb band, as indicated with a solid circle in Figure 2.

To indentify the genomic counterpart of the functional heavy chain gene, both λV_{H}7.4E and λV_{H}3.8E were digested with PstI and hybridized with the mixed V_{H}1 and V_{H}2 probes derived from the cDNA clone, pM γ2a-1. λV_{H}7.4E contained two PstI fragments at 300 bp and 130 bp. Since the V_{H}1 probe contained sequences encoding for the amino acid residue -5 which is just upstream of the intron/exon boundary within the leader peptide, the difference between the longer PstI fragment in the genomic clone (200 bp) and that in the cDNA clone (228 bp) suggested the size of the intron between the leader peptide and the variable region gone to be 70 bp. Clone λV_{H}3.8E contained different length PstI fragments and did not cross-hybridize with V_{H}1 and V_{H}2 probes. To further verify that λV_{H}7.4E is the genomic counterpart of the cDNA clone pM γ2a-1, a 24-mer oligonucleotide probe corresponding to nucleotide 352 to 375 (Figure 1B) was synthesized and found to specifically hybridize to the 130 bp PstI fragment of the λv_{H}7.4E (data not shown). This oligomer contained sequences encoding for the CDR3 region and should be characteristic of this V gene. λV_{H}7.4E was used in the construction of the mouse/ human chimeric heavy chain gene.

### Vectors and expression system

The light and heavy chain V genes cloned from the 17-1A cells were joined to human K and γ3 constant region genes in expression vectors pSV184 Hneo (Chang, A. C. Y., and S. N. Cohn, J. Bacteriol., (1978) 143:1141) and pSV2 Hgpt (Mulligan, R. C., and P. Berg., Proc. Natl. Acad. Sci. USA (1981) 78:2072), respectively. The pSV184ΔHneo-DNSV_{L}-hC_{K} plasmid was used which had the dansyl-specific light chain gene and the human constant region K gene cloned into the BamHI and HindIII sites in pSV184 Hneo. To construct the desired chimeric gene, the HindIII fragment of pSV184Δ HneoDNSV_{L} -hC_{K} containing the dansyl V_{L} gene was replaced with the 4.2 kb HindIII fragment containing the functionally rearranged genomic light chain gene of 17-1A derived from the clone pV_{K} 4.2H. For the heavy chain vector, the EcoRI fragment in the pSV2Δ HgptDNSV_{H}-hC_{γ3} plasmid containing the dansyl-specific V_{H} gene was replaced with the 7.4 kb EcoRI fragment containing the functionally rearranged 17-1A heavy chain gene derived from the genomic clone λV_{H}7.4E. The resulting plasmid is designated as pSV₂ΔHgpt171AV_{H}-hC_{γ3}. The structure of pSV184Δ Hneo17-1AV_{K}-hC_{K} is shown in Figure 3A and the structure of pSV2ΔHgpt17-1AV_{H}-hC_{γ3} in Figure 3B. (Immunoglobulin DNA is represented by thick lines and exons by solid boxes. Vector sequences are shown as thin lines. The transcriptional direction of the neo and gpt genes are as indicated. The ranges of the DNA sequences art indicated with two-way horizontal arrows. L, leader exon; V, VJ or VDJ exon; C, constant region exon; H, hinge exon; 1, 2, and 3, exons encoding other domains of the heavy chain gene. Restriction endonucleases abbreviations: E, EcoRI; B, BamHI; H, HindIII).

The light and heavy chain vectors shown in Figure 3 were used to transfect mouse myeloma cells. The pACYC184 plasmid confers chloramphenicol resistance and the pBR plasmid confers ampicillin resistance. It is therefore possible to transform E. coli with both light and heavy chain plasmids and select cells expressing dual drug resistance phenotypes. To transfect the chimeric genes into mouse myeloma cells, E. coli harboring both plasmids were converted to protoplasts and fused with a nonproducing mouse myeloma cell line, Sp2/0. After protoplast fusion, the transfected cells were initially selected only for neo gene activity in the presence of G418. The stable transformant lines were subsequently carried in medium containing both G418 and mycophenolic acid.

### Analysis of chimeric antibody production.

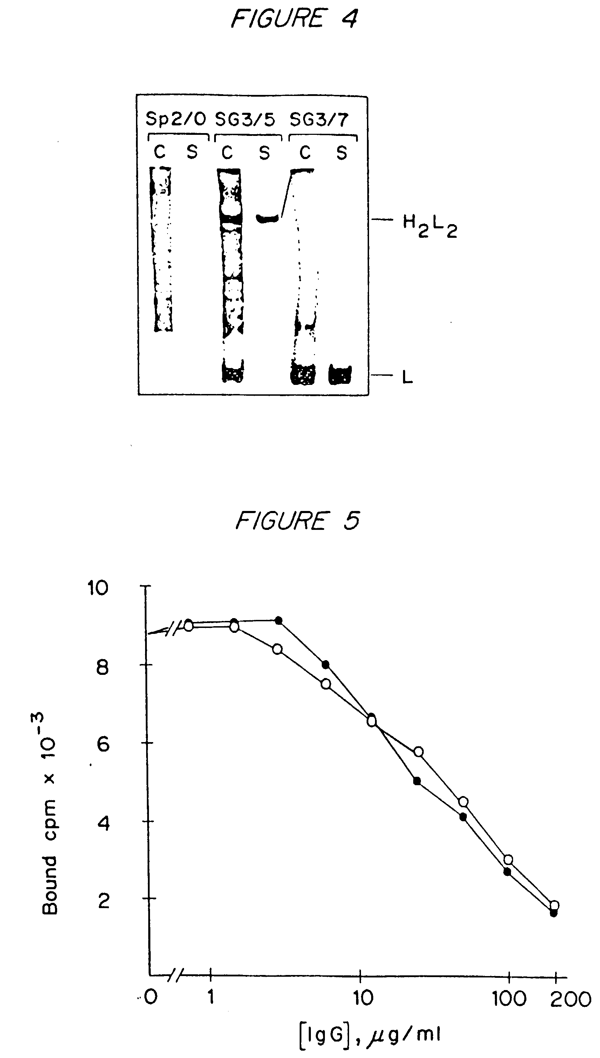

To examine heavy and light chain protein synthesis, transfected cells were labeled with ³⁵S-methionine for three hours, after which both the cytoplasmic extract and secreted material were immunoprecipitated with Sepharose-bound goat anti-human K antibody. The precipitated material was analyzed on 5% SDS/polyacrylamide gels containing phosphate buffer under nonreducing conditions Matsuuchi, L. et al. 1981 Biochem 20:4827. From 4 x10⁶ cells, seven stable transformants were established and analyzed. Two of these clones produced both heavy and light chain proteins, two produced only light chain proteins, and three did not produce any detectable amounts of immunoglobulin proteins. Figure 4 shows the result of a biosynthetic labeling experiment for the SG3/5 cell line that produced both heavy and light chain protein. (c, cytoplasmic; s, secreted. The positions of the tetrameric H₂L₂ protein and the light chain (L) protein are indicated.) A mixture of heavy and light chain molecules were detected in the cell extreact; however, the major components secreted into the culture medium were the tetrameric molecules, H₂L₂. Similar results were obtained using the Sepharose-bound anti-human IgG Fc antibody for the immunoprecipitations. The SG3/7 cells which also showed resistance to both G418 and mycophenolic acid produced and secreted the light chain proteins only.

### Assay of chimeric antibody.

The antibody secretion level of the SG3/5 cells was estimated to be 20 µg/ml by particle concentration fluorescence immunoassay. The culture supernatant was used as a source of chimeric MAb SG3/5 without further purification. A binding inhibition assay was used to demonstrate that the chimeric MAb SG3/5 bound to the same surface antigen of the SW1116 colorectal carcinoma cells as MAb 17-1A. As shown in Figure 5, the binding curves of radioiodinated 17-1A and SG3/5 were superimposable. This indicated that the different constant regions of MAb 17-1A and SG3/5 had little effect on the antigen-binding properties of these two antibodies.

### Example 2. Production of chimeric IgG1, 2 and 4.

The DNA segment encoding the variable region of the heavy chain from from the mouse MAb 17-1A was linked to the human γ1, γ2, and γ4 constant regions. The resulting chimeric heavy chain genes were cotransfected with the chimeric light chain gene (Figure 3B) into Sp2/0 cells to generate stable cell lines secreting (γ1,κ), (γ2,κ) and (γ4,κ) antibodies. All these chimeric antibodies were shown to retain the same tumor antigen-binding specificity as the murine MAb 17-1A.

### Example 3. Production of Chimeric 17-1A IgM.

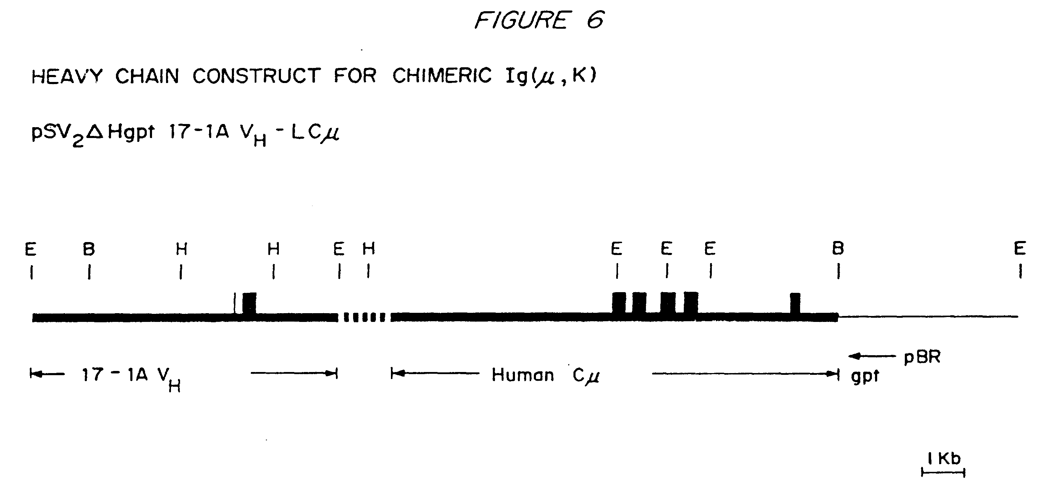

The chimeric µ construct (psV2ΔHgpt17-1AV_{H}-hC_{µ}) is shown in Figure 6. This gene construct, together with the 17-1A chimeric K light chain gene construct (fig 3A), were transfected into the non-producing mouse myeloma Sp2/0 cells. A stable cell line producing chimeric Ig(µ,κ) antibody was established. Secreted antibody was purified on an anti-human IgM affinity column. The purified IgM had similar HPLC profiles as a human pentameric IgM control with 99% of the material under one peak. This purified chimeric IgM was used to check the binding specificity and complement-dependent cytotoxicity.

### Competitive inhibition of ¹²⁵I 17-1A binding to the HT-29 cell line by chimeric 17-1A IgG₁ and IgM.

¹²⁵I-labeled native 17-1A was incubated with adherent HT-29 colon cancer cells in the presence or absence of varying concentrations of competing non-radioactive chimeric 17-1A IgG₁ and IgM for 2 hours at 4°C. Cell-associated radioactivity was determined. The inhibition curve for each competing immunoglobulin preparation was plotted and the concentration producing 50% inhibition (I₅₀) determined.

**TABLE 1**

| Antibody | ID₅₀ |
|---|---|
| 17-1A IgG₁ | 2.8 |
| 17-1A IgM | 4.5 |

### Complement-dependent cytotoxicity of tumor and normal cell lines mediated by chimeric 17-1A IgG₁ and IgM.

Complement dependent cytotoxicity was assessed by a 45 minute radiolabel release assay of antibody coated target cells incubated with serial dilutions of rabbit complement. Target cells employed in the assay were the human colorectal carcinoma lines, SW1116, HT-29, and SW948; human breast carcinoma lines, BT-20 and ZR75-1; human ovarian carcinoma, OVGAR3; human embryonic kidney (HEK); and human embryonic intestine (HEI).

**TABLE 2**

| Rabbit Compelement Dilution | % 51-Cr release with 17-1A IgM against: | | | | | | | |
|---|---|---|---|---|---|---|---|---|
| | SW1116 | HT-29 | SW948 | BT-20 | ZR75-1 | OVCAR3 | HEK | HEI |
| 1/2 | 89 | 75 | 93 | 44 | 23 | 28 | 11 | 6 |
| 1/4 | 56 | 62 | 68 | 20 | 7 | 18 | 8 | 4 |
| 1/8 | 47 | 56 | 27 | 5 | 0 | 6 | 0 | 0 |
| 1/16 | 33 | 9 | 10 | 0 | - | 0 | - | - |
| 1/32 | 0 | 0 | 4 | - | - | - | - | - |

**TABLE 3**

| Rabbit Compelement Dilution | % 51-Cr release with 17-1A IgG₁ against: | | | | | | | |
|---|---|---|---|---|---|---|---|---|
| | SW1116 | HT-29 | SW948 | BT-20 | ZR75-1 | OVCAR3 | HEK | HEI |
| 1/2 | 12 | 10 | 12 | 9 | 0 | 5 | 3 | 0 |
| 1/4 | 5 | 0 | 0 | 0 | - | 0 | 0 | - |
| 1/8 | 0 | - | - | - | - | - | - | - |
| 1/16 | - | - | - | - | - | - | - | - |
| 1/32 | - | - | - | - | - | - | - | - |

### Example 4. In vitro and in vivo studies demonstrating anti-tumor activity of chimeric 17-1A antibodies.

### A. In Vitro Studies

The isolated G1K 17-1A chimeric gene product was biologically tested in 1) antibody-dependent cell-mediated cytotoxicity assays (ADCC) and 2) binding assays using HT-29 cells (colon cancer cell line that expresses the 17-1A antigen) and partially purified 17-1A antigen. Methodologies for these investigations are presented in detail in Shaw, D.R. et al. J. Immunol., June 1987; Levy, P.C. et al. J. Immunol, 123(2): 594(1979); and Ross, A.H. et al. Biochem. Biophys. Res. Commun. 135(1): 297 (1986). In the ADCC and competitive binding studies in HT-29 cells, G1K 17-1A Chimeric was tested in conjunction with the native murine 17-1A IgG and 3 additional chimeric isotypes shown below; rabbit anti-CEM* was employed as a polyclonal control. In the competitive binding studies utilizing partially purified 17-1A antigen, G1K 17-1A Chimeric was compared to the native murine 17-1A IgG.

The data for the monocyte and lymphocyte ADCC assays were generated from four separate experiments using monocytes and lymphocytes from four different normal adult donors. Mean spontaneous release ± SD was 18.7 ± 2.8% of total target cell ⁵¹CR (N=28).

The monocyte ADCC assay was performed utilizing Effector: Target (E:T) ratios of 25:1, 10:1 and 3:1. The mean % lysis (± S.D) for each test material at the specified E:T ratios are presented below.

**TABLE 4**

| Antibody | E:T Ratio | | |
|---|---|---|---|
| | 25:1 | 10:1 | 3:1 |
| None | 3.5 (±1.1) | 1.5 (±1.4) | 1.0 (±0.9) |
| 17-1A | 10.8 (±2.2) | 7.2 (±2.2) | 3.6 (±1.4) |
| G1K Chimeric | 11.8 (±4.9) | 6.0 (±1.8) | 2.0 (±0.6) |
| G2K Chimeric | 4.0 (±1.9) | 2.8 (±0.5) | 1.2 (±1.0) |
| G3K Chimeric | 12.4 (±7.9) | 8.1 (±7.1) | 2.5 (±0.4) |
| G4K Chimeric | 4.9 (±2.8) | 2.8 (±1.6) | 1.2 (±1.2) |
| Rabbit anti-CEM* | 29.7 (±13.0) | 18.8 (±7.8) | 8.9 (±1.0) |

| | | | |
|---|---|---|---|
| *Polyclonal rabbit anti-sera against a T cell lymphoblastoid cell line. | | | |

In the monocyte ADCC assay the % lysis with G1K 17-1A chimeric IgG was comparable to that seen with murine 17-1A.

The lympocyte ADCC assay was performed utilizing E:T ratios of 100:1, 30A1 and 10:1. The mean % lysis (±S.D) values are shown below.

**TABLE 5**

| Antibody | E:T Ratio | | |
|---|---|---|---|
| | 100:1 | 30:1 | 10:1 |
| None | 5.6 (±0.5) | 2.6 (±1.9) | 1.4 (±1.8) |
| 17-1A | 33.6 (±9.5) | 15.7 (±8.6) | 7.3 (±4.8) |
| G1K Chimeric | 34.5 (±5.2) | 19.9 (±9.8) | 10.0 (±4.2) |
| G2K Chimeric | 11.5 (±2.6) | 3.2 (±3.0) | 1.7 (±1.8) |
| G3K Chimeric | 30.7 (±8.5) | 19.2 (±11.3) | 11.8 (±8.8) |
| G4K Chimeric G4k | 11.4 (±2.8) | 4.6 (±2.8) | 1.8 (±1.9) |
| Rabbit anti-CEM | 60.0 (±7.5) | 42.9 (±12.1) | 23.0 (±13.5) |

In the lymphocyte ADCC assay the % lysis with G1K 17-1A Chimeric IgG was comparable to that seen with 17.1A.

In the binding assay utilizing intact HT-29 cells, the cells were coated with the test antibody. Quantitation for the 17-1A and rabbit anti-CEM used ¹²⁵I-protein A; quantitation for chimeric antibodies used ¹²⁵I-anti-human IgG(Fc). Data on the quantitation of IgG bound to the tumor cells are presented below. The results represent a mean (±S.E.) of four or five separate experiments.

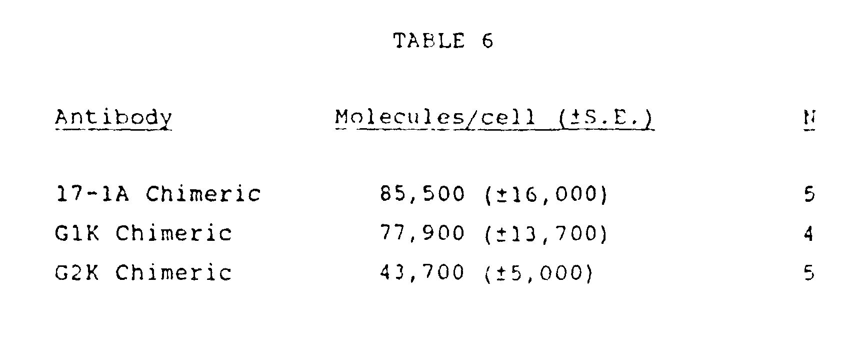

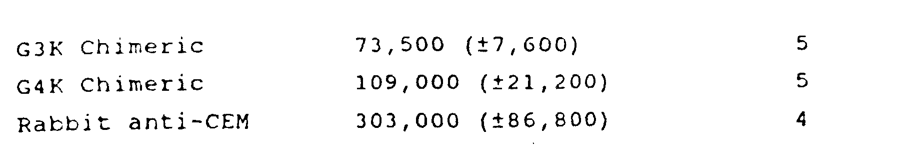

Einding to the HT-29 cells was comparable between G1K 17-1A Chimeric IgG murine 17-1A.

In the competitive binding assay using partially purified 17-1A antigen, microtiter plates were incubated with partially purified 17-1A antigen (isolated from SW116 cells) for 2 hours at 37°C. Non-specific binding sites of the wells were blocked with PBS + 1% BSA for 1 hour at room temperature and were washed with PBS. ¹²⁵I labeled murine 17-1A and the competing antibody (cold murine 17-1A or G1K 17-1A Chimeric IgG) were incubated in the wells at 4°C overnight. The wells were washed 3 times with PBS and counted in a gamma counter. The 50% binding values for G1K 17-1A Chimeric and murine 17-1A were determined to be 0.30 µg/ml and 0.16 µg/ml, respectively. These results indicate that the G1K 17-1A Chimeric IgG is comparable to murine 17-1A in antibody binding.

In addition to the above in-vitro studies, in vitro immunohistopathology studies with G1K 17-1A and murine 17-1A were performed on various human tumor tissues using immunofluorescence technology. Murine 17-1A (24 µg/ml) and G1K 17-1A Chimeric IgG (96 µg/ml) were shown to react in 4 out of 4 colon tumor specimens examined. Other tumor cells examined included pancreatic cancer, lung cancer (adeno- and bronchia-) breast fibroadenomas and carcinomas, stomach cancer, melanoma, hepatoma and ovarian cancer. Murine 17-1A reacted with lung cancer (bronchia), stomach cancer, melanoma and ovarian cancer. G1K 17-1A Chimeric IgG did not react with any of the tumor specimens examined other than the colon tumors.

### B. In Vivo Studies

Tumor growth inhibition experiments in nude mice have indicated that G1K 17-1A Chimeric is active in retarding tumor enlargement. The nude mouse tumor inhibition study was performed in 6 to 8 weeks old nude mice (NU/NU Balb C background) using previously described methods (Herlyn, D.M. et al., Cancer Res. 40:717 (1980)). Mice (5/group) were injected subcutaneously in the neck with 5 x 10⁶ SW948 (colon tumor cells) and then injected intra-perioneally with 5 daily doses of 100 µg of murine 17-1A or each of 4 chimeric isotopes (including G1K 17-1A) or a control anti-influrenza antibody(H24B5). Tumor size was monitored weekly for 6 weeks by external measurement. The individual animal tumor volumes and group mean values for each measurement interval are shown in the Table 7 on the following page. The relative tumor inhibition ranking was found to be 17-1A G4K G1K. The chimeric antibodies G3K and G2K were not effective at the dose level tested.

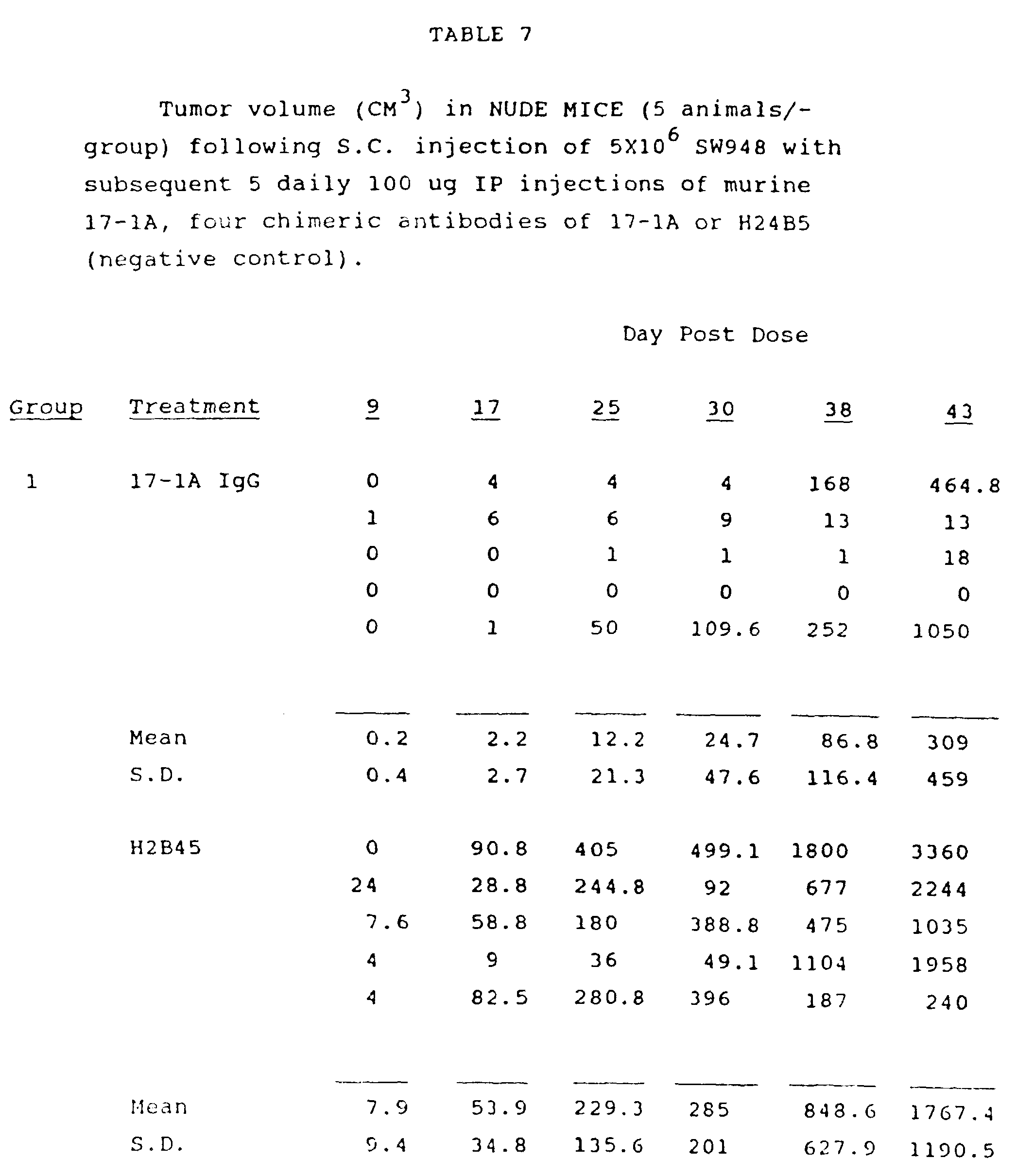

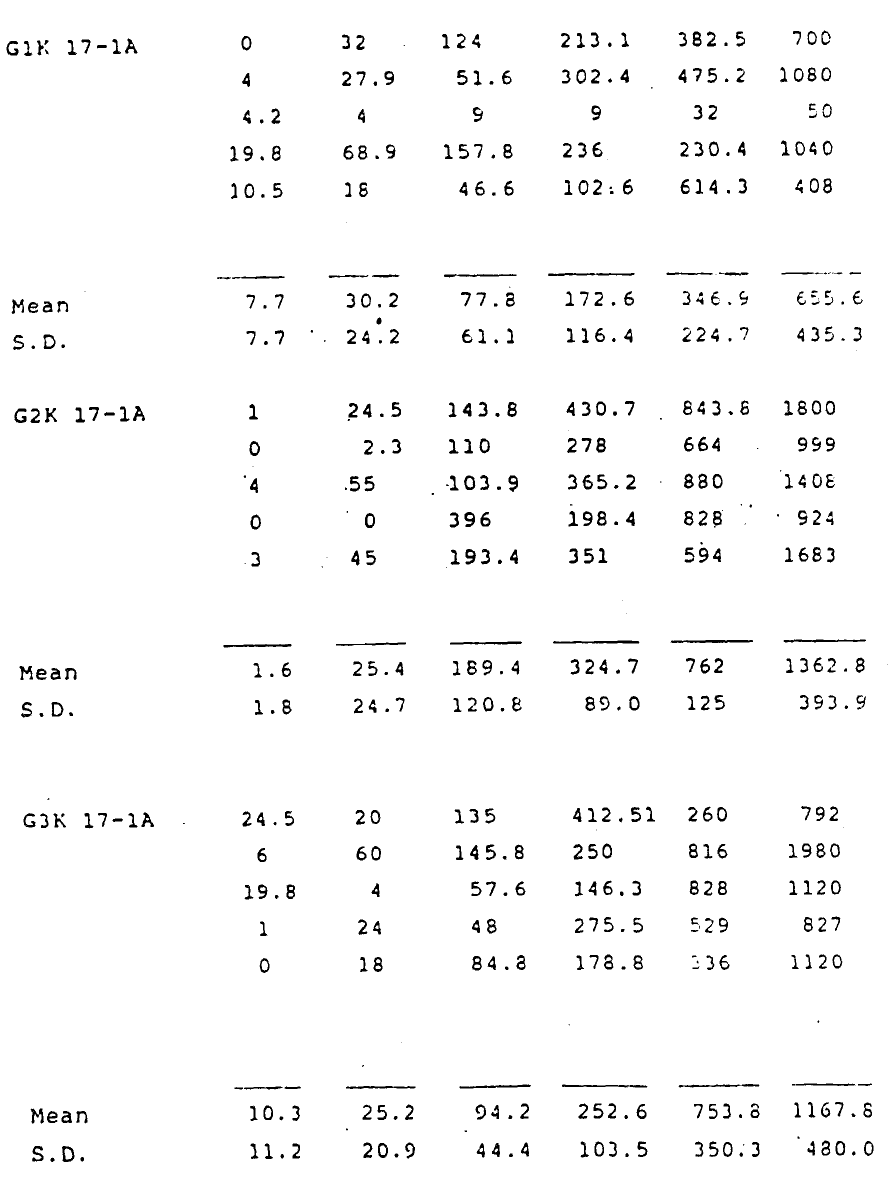

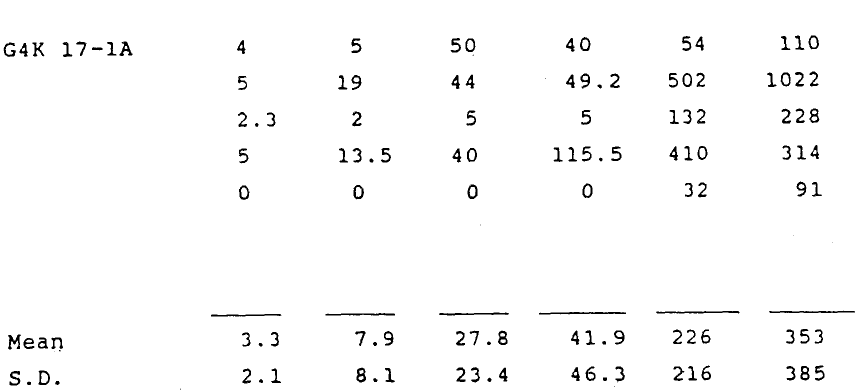

## Claims (Claims for the following Contracting State(s): BE, CH, DE, FR, GB, IT, LI, LU, NL, SE)

1. A murine/human chimaeric immunoglobulin or chimaeric fragment thereof, comprising an antigen binding region derived from a murine (e.g. mouse) antibody specific for the antigen defined by the 17-1A murine monoclonal antibody linked to at least a portion of a constant region of human origin, the chimaeric immunoglobulin or immunoglobulin fragment retaining binding specificity for said antigen.

2. A chimaeric immunoglobulin, comprising:
a) at least one chimaeric heavy chain comprising an antigen-binding region derived from the heavy chain of a murine antibody specific for the antigen defined by the 17-1A murine monoclonal antibody linked to a human heavy chain constant region (e.g. of the gamma type) in association with;
b) at least one chimaeric light chain comprising an antigen-binding region derived from a light chain of the murine antibody linked to a human light chain constant region; the chimaeric immunoglobulin retaining binding specificity for the antigen defined by the 17-1A murine monoclonal antibody.

3. A chimaeric immunoglobulin, comprising:
a) at least one chimaeric heavy chain comprising an antigen-binding region derived from the heavy chain of a murine antibody specific for the antigen defined by the 17-1A murine monoclonal antibody linked to a human heavy chain constant region (e.g. of the gamma type) in association with;
b) at least one chimaeric light chain comprising an antigen binding region derived from a light chain of the murine antibody linked to a human light chain constant region; and
c) a nonimmunoglobulin protein or peptide (said protein or peptide being for example an anti-cancer therapeutic agent, a cytotoxic agent, a metal binding agent, or a functional domain thereof) linked to at least one of the human constant regions; the chimaeric immunoglobulin retaining binding specificity for the antigen defined by the 17-1A murine monoclonal antibody.

4. A chimaeric Fab, Fab' or F(ab')₂ immunoglobulin fragment, comprising:
a) antigen binding regions of murine origin specific for the antigen defined by the 17-1A murine monoclonal antibody; and
b) a human constant region; the chimaeric immunoglobulin retaining binding specificity for said antigen.

5. A reagent for tumour scintigraphy, comprising the chimaeric immunoglobulin fragment according to claim 4, labelled with a radioisotope selected from the group consisting of ¹³¹ Iodine, ¹²⁵ Iodine, ^{99m} Technetium, and ¹¹¹ Indium.

6. A chimaeric immunoglobulin fragment according to claim 4 further comprising:
c) a metal binding protein (e.g. metallothionein) or protein domain linked to the constant region.

7. A fused gene encoding a chimaeric murine/human immunoglobulin specific for the antigen defined by the 17-1A murine monoclonal antibody comprising:
a) a first DNA sequence encoding at least the functional portion of a variable region of a murine immunoglobulin chain linked to:
b) a second DNA sequence encoding at least a portion of a constant region of a human immunoglobulin.

8. A fused gene according to claim 7, further comprising:
c) a third DNA sequence encoding a nonimmunoglobulin protein or peptide (said protein or peptide being for example an anti-cancer therapeutic agent, a cytotoxic agent, a metal binding agent, or a functional domain thereof) the sequence linked to the 3' end of the second DNA sequence.

9. A chimaeric immunoglobulin or immunoglobulin fragment according to claim 1, or a fragment according to claim 4, for use in therapy or diagnosis

10. A chimaeric immunoglobulin according to claim 1, for use in the immunotherapy of a gastrointestinal tumour.

11. A chimaeric immunoglobulin for use in tumour immunotherapy comprising:
a) at least one chimaeric heavy chain the comprising an antigen-binding region derived from the heavy chain of a murine antibody specific for the antigen defined by the 17-1A murine monoclonal antibody linked to a human heavy chain constant region in association with;
b) at least one chimaeric light chain comprising an antigen-binding region derived from a light chain of a murine antibody linked to a human light chain constant region; and
c) an anti-tumour therapeutic or cytotoxic agent linked to at least one of the constant regions.

12. Use of a chimaeric immunoglobulin or immunoglobulin fragment according to claim 1, a fragment according to claim 4, or a fused gene according to claim 7, for the manufacture of a medicament or diagnostic agent e.g. for use in tumour immunotherapy or tumour diagnosis.

## Claims (Claims for the following Contracting State(s): AT, ES, GR)

1. A method for preparing a murine/human chimeric immunoglobulin or chimeric fragment thereof, comprising an antigen binding region derived from a murine (e.g. mouse) antibody specific for the antigen defined by the 17-1A murine monoclonal antibody linked to at least a portion of a constant region of human origin, the chimeric immunoglobulin or immunoglobulin fragment retaining binding specificity for said antigen, the method comprising the step of preparing, for each of the light and heavy chain components of the chimeric immunoglobulin, a fused gene comprising a first DNA segment which encodes at least the functional portion of the murine variable region linked to a second DNA segment encoding at least a part of a human constant region.

2. A method for preparing a chimeric immunoglobulin, comprising:
(a) at least one chimeric heavy chain comprising an antigen-binding region derived from the heavy chain of a murine antibody specific for the antigen defined by the 17-1A murine monoclonal antibody linked to a human heavy chain constant region (e.g. of the gamma type) in association with:
(b) at least one chimeric light chain comprising an antigen-binding region derived from a light chain of the murine antibody linked to a human light chain constant region; the chimeric immunoglobulin retaining binding specificity for the antigen defined by the 17-1A murine monoclonal antibody, the method comprising the step of preparing, for each of the light and heavy chain components of the chimeric immunoglobulin, a fused gene comprising a first DNA segment which encodes at least the functional portion of the murine variable region linked to a second DNA segment encoding at least a part of a human constant region.

3. A method for preparing a chimeric immunoglobulin, comprising:
(a) at least one chimeric heavy chain comprising an antigen-binding region derived from the heavy chain of a murine antibody specific for the antigen defined by the 17-1A murine monoclonal antibody linked to a human heavy chain constant region (e.g. of the gamma type) in association with:
(b) at least one chimeric light chain comprising an antigen-binding region derived from a light chain of the murine antibody linked to a human light chain constant region; and
(c) a nonimmunoglobulin protein or peptide (said protein or peptide being for example an anti-cancer therapeutic agent, a cytotoxic agent, a metal binding agent, or a functional domain thereof) linked to at least one of the human constant regions; the chimeric immunoglobulin retaining binding specificity for the antigen defined by the 17-1A murine monoclonal antibody, the method comprising the step of preparing, for each of the light and heavy chain components of the chimeric immunoglobulin, a fused gene comprising a first DNA segment which encodes at least the functional portion of the murine variable region linked to a second DNA segment encoding at least a part of a human constant region.

4. The method of claim 1 wherein the chimeric fragment is a chimeric Fab, Fab' or F(ab')₂ immunoglobulin fragment, comprising:
(a) antigen binding regions of murine origin specific for the antigen defined by the 17-1A murine monoclonal antibody; and
(b) a human constant region; the chimeric immunoglobulin retaining binding specificity for said antigen.

5. A method for producing a reagent for tumour scintigraphy, comprising the step of labelling the chimeric immunoglobulin fragment as defined in claim 4 with a radioisotope selected from the group consisting of ¹³¹ Iodine, ¹²⁵ Iodine, ^{99m} Technetium, and ¹¹¹ Indium.

6. The method of claim 4 wherein the chimeric immunoglobulin fragment further comprises:
(c) a metal binding protein (e.g. metallothionein) or protein domain linked to the constant region.

7. A method for making a fused gene encoding a chimeric murine/human immunoglobulin specific for the antigen defined by the 17-1A murine monoclonal antibody comprising the step of linking:
a) a first DNA sequence encoding at least the functional portion of a variable region of a murine immunoglobulin chain, to:
b) a second DNA sequence encoding at least a portion of a constant region of a human immunoglobulin.

8. The method of claim 7 wherein the fused gene further comprises:
c) a third DNA sequence encoding a nonimmunoglobulin protein or peptide (said protein or peptide being for example an anti-cancer therapeutic agent, a cytotoxic agent, a metal binding agent, or a functional domain thereof) the sequence linked to the 3' end of the second DNA sequence.

9. A chimeric immunoglobulin or immunoglobulin fragment as defined in claim 1, or a fragment as defined in claim 4, for use in therapy or diagnosis.

10. A chimeric immunoglobulin as defined in claim 1, for use in the immunotherapy of a gastrointestinal tumour.

11. A chimeric immunoglobulin for use in tumour immunotherapy comprising:
a) at least one chimeric heavy chain comprising an antigen-binding region derived from the heavy chain of a murine antibody specific for the antigen defined by the 17-1A murine monoclonal antibody linked to a human heavy chain constant region in association with:
b) at least one chimeric light chain comprising an antigen-binding region derived from a light chain of a murine antibody linked to a human light chain constant region; and
c) an anti-tumour therapeutic or cytotoxic agent linked to at least one of the constant regions.

12. Use of a chimeric immunoglobulin or immunoglobulin fragment as defined in claim 1, a fragment as defined in claim 4, or a fused gene as defined in claim 7, for the manufacture of a medicament or diagnostic agent e.g. for use in tumour immunotherapy or tumour diagnosis.

## Patentansprüche (Patentansprüche für folgende(n) Vertragsstaat(en): BE, CH, DE, FR, GB, IT, LI, LU, NL, SE)

1. Ein chimäres Murin/Mensch-Immunoglobulin oder ein chimäres Fragment davon, das eine von einem murinen (z.B. Maus) Antikörper stammende Antigen-Bindungsregion aufweist, die spezifisch für das von dem murinen monoklonalen Antikörper 17-1A definierte Antigen ist und mindestens mit einem Teil einer konstanten Region menschlichen Ursprungs verknüpft ist, wobei das chimäre Immunoglobulin oder Immunoglobulin-Fragment eine Bindungsspezifität für besagtes Antigen beibehält.

2. Ein chimäres Immunoglobulin, das umfaßt:
a) mindestens eine chimäre schwere Kette, die eine von der schweren Kette eines murinen Antikörpers stammende Antigen-Bindungsregion aufweist, die spezifisch für das von dem murinen monoklonalen Antikörper 17-1A definierte Antigen ist, verknüpft mit einer menschlichen konstanten Region der schweren Kette (z. B. des gamma Typs) und assoziiert damit:
b) mindestens eine chimäre leichte Kette, die eine von einer leichten Kette des murinen Antikörpers stammende Antigen-Bindungsregion aufweist, die mit einer menschlichen konstanten Region der leichten Kette verknüpft ist, wobei das chimäre Immunoglobulin eine Bindungsspezifität für das von dem murinen monoklonalen Antikörper 17-1A definierte Antigen beibehält.

3. Ein chimäres Immunoglobulin, das umfaßt:
a) mindestens eine chimäre schwere Kette, die eine von der schweren Kette eines murinen Antikörpers stammende Antigen-Bindungsregion aufweist, die spezifisch für das von dem murinen monoklonalen Antikörper 17-1A definierte Antigen ist, verknüpft mit einer menschlichen konstanten Region der schweren Kette (z. B. des gamma Typs) und assoziiert damit:
b) mindestens eine chimäre leichte Kette, die eine von einer leichten Kette des murinen Antikörpers stammende Antigen-Bindungsregion aufweist, die mit einer menschlichen konstanten Region der leichten Kette verknüpft ist, und
c) ein Nicht-Immunoglobulin-Protein oder Peptid (besagtes Protein oder Peptid ist beispielsweise ein therapeutisches Mittel gegen Krebs, ein cytotoxisches Mittel, ein metallbindendes Mittel oder eine funktionale Domäne davon), das mit mindestens einer der menschlichen konstanten Regionen verknüpft ist, wobei das chimäre Immunoglobulin eine Bindungsspezifität für das von dem murinen monoklonalen Antikörper 17-1A definierte Antigen beibehält.

4. Ein chimäres Fab, Fab' oder F(ab')₂ Immunoglobulin-Fragment, das umfaßt:
a) Antigen-Bindungsregionen murinen Ursprungs, die spezifisch für das von dem murinen monoklonalen Antikörper 17-1A definierte Antigen sind, und
b) eine menschliche konstante Region, wobei das chimäre Immunoglobulin eine Bindungsspezifität für besagtes Antigen beibehält.

5. Ein Reagenz zur Tumor-Szintigraphie, das das chimäre Immunoglobulin-Fragment gemäß Anspruch 4 umfaßt und mit einem Radioisotop markiert ist, das aus der Gruppe gewählt ist, die aus ¹³¹Jod, ¹²⁵Jod, ^{99m}Technetium und ¹¹¹Indium besteht.

6. Ein chimäres Immunoglobulin-Fragment gemäß Anspruch 4, das außerdem umfaßt:
c) ein metallbindendes Protein (z. B. Metallothionein) oder eine mit der konstanten Region verknüpfte Protein-Domäne.

7. Ein fusioniertes Gen, das ein chimäres Murin/Mensch-Immunoglobulin codiert, das spezifisch für das von dem murinen monoklonalen Antikörper 17-1A definierte Antigen ist, das umfaßt:
a) eine erste DNS-Sequenz, die mindestens den funktionalen Teil einer variablen Region einer murinen Immunoglobulin-Kette codiert und verknüpft damit:
b) eine zweite DNS-Sequenz, die mindestens einen Teil einer konstanten Region eines menschlichen Immunoglobulins codiert.

8. Ein fusioniertes Gen gemäß Anspruch 7, das außerdem umfaßt:
c) eine dritte DNS-Sequenz, die ein Nicht-Immunoglobulin-Protein oder Peptid (besagtes Protein oder Peptid ist beispielsweise ein therapeutisches Mittel gegen Krebs, ein cytotoxisches Mittel, ein metallbindendes Mittel oder eine funktionale Domäne davon) codiert, wobei die Sequenz mit dem 3' Ende der zweiten DNS-Sequenz verknüpft ist.

9. Ein chimäres Immunoglobulin oder Immunoglobulin-Fragment gemäß Anspruch 1 oder ein Fragment gemäß Anspruch 4 zur Verwendung in Therapie oder Diagnose.

10. Ein chimäres Immunoglobulin gemäß Anspruch 1 zur Verwendung in der Immuntherapie eines gastrointestinalen Tumors.

11. Ein chimäres Immunoglobulin zur Tumor-Immuntherapie, das umfaßt:
a) mindestens eine chimäre schwere Kette, die eine von der schweren Kette eines murinen Antikörpers stammende Antigen-Bindungsregion aufweist, die spezifisch für das von dem murinen monoklonalen Antikörper 17-1A definierte Antigen ist, verknüpft mit einer konstanten Region der menschlichen schweren Kette und assoziiert damit:
b) mindestens eine chimäre leichte Kette, die eine von einer leichten Kette eines murinen Antikörpers stammende Antigen-Bindungsregion aufweist, die mit einer konstanten Region der menschlichen leichten Kette verknüpft ist; und
c) ein Antitumor-Therapeutikum oder cytotoxisches Mittel, das mit mindestens einer der konstanten Regionen verknüpft ist.

12. Verwendung eines chimären Immunoglobulins oder Immunoglobulin-Fragments gemäß Anspruch 1, eines Fragments gemäß Anspruch 4 oder eines fusionierten Gens gemäß Anspruch 7 zur Herstellung eines Medikaments oder diagnostischen Mittels, z. B. zur Tumor-Immuntherapie oder Tumor-Diagnose.

## Patentansprüche (Patentansprüche für folgende(n) Vertragsstaat(en): AT, ES, GR)

1. Ein Verfahren zur Herstellung eines chimären Murin/Mensch-Immunoglobulins oder chimären Fragments davon, das eine von einem murinen (z.B. Maus) Antikörper stammende Antigen-Bindungsreaktion aufweist, die spezifisch für das von dem murinen monoklonalen Antikörper 17-1A definierte Antigen ist und mindestens mit einem Teil einer konstanten Region menschlichen Ursprungs verknüpft ist, wobei das chimäre Immunoglobulin oder Immunoglobulin-Fragment eine Bindungsspezifität für besagtes Antigen beibehält, wobei das Verfahren für jede der Komponenten des chimären Immunoglobulins, der leichten und schweren Kette, den Schritt der Herstellung eines fusionierten Gens umfaßt, das einen ersten DNS-Abschnitt umfaßt, der mindestens den funktionalen Teil der murinen variablen Region codiert und mit einem zweiten DNS-Abschnitt verknüpft ist, der mindestens einen Teil einer menschlichen konstanten Region codiert.

2. Ein Verfahren zur Herstellung eines chimären Immunoglobulins, das umfaßt:
(a) mindestens eine chimäre schwere Kette, die eine von der schweren Kette eines murinen Antikörpers stammende Antigen-Bindungsregion aufweist, die spezifisch für das von dem murinen monoklonalen Antikörper 17-1A definierte Antigen ist, verknüpft mit einer menschlichen konstanten Region einer schweren Kette (z. B. des gamma Typs) und assoziiert damit:
(b) mindestens eine chimäre leichte Kette, die eine von einer leichten Kette des murinen Antikörpers stammende Antigen-Bindungsregion aufweist, die mit einer menschlichen konstanten Region der leichten Kette verknüpft ist, wobei das chimäre Immunoglobulin eine Bindungsspezifität für das von dem murinen monoklonalen Antikörper 17-1A definierte Antigen beibehält, wobei das Verfahren für jede der Komponenten des chimären Immunoglobulins, der leichten und schweren Kette, den Schritt der Herstellung eines fusionierten Gens umfaßt, das einen ersten DNS-Abschnitt umfaßt, der mindestens den funktionalen Teil der murinen variablen Region codiert und mit einem zweiten DNS-Abschnitt verknüpft ist, der mindestens einen Teil einer menschlichen konstanten Region codiert.

3. Ein Verfahren zur Herstellung eines chimären Immunoglobulins, das umfaßt:
(a) mindestens eine chimäre schwere Kette, die eine von der schweren Kette eines murinen Antikörpers stammende Antigen-Bindungsregion aufweist, die spezifisch für das von dem murinen monoklonalen Antikörper 17-1A definierte Antigen ist, verknüpft mit einer menschlichen konstanten Region der schweren Kette (z. B. des gamma Typs) und assoziiert damit:
(b) mindestens eine chimäre leichte Kette, die eine von einer leichten Kette des murinen Antikörpers stammende Antigen-Bindungsregion aufweist, die mit einer menschlichen konstanten Region der leichten Kette verknüpft ist; und
(c) ein Nicht-Immunoglobulin-Protein oder Peptid (besagtes Protein oder Peptid ist beispielsweise ein therapeutisches Mittel gegen Krebs, ein cytotoxisches Mittel, ein metallbindendes Mittel oder eine funktionale Domäne davon), das mit mindestens einer der menschlichen konstanten Regionen verknüpft ist, wobei das chimäre Immunoglobulin eine Bindungsspezifität für das von dem murinen monoklonalen Antikörper 17-1A definierte Antigen beibehält, wobei das Verfahren für jede der Komponenten des chimären Immunoglobulins, der leichten und schweren Kette, den Schritt der Herstellung eines fusionierten Gens umfaßt, das einen ersten DNS-Abschnitt umfaßt, der mindestens den funktionalen Teil der murinen variablen Region codiert und mit einem zweiten DNS-Abschnitt verknüpft ist, der mindestens einen Teil einer menschlichen konstanten Region codiert.

4. Das Verfahren nach Anspruch 1, worin das chimäre Fragment ein chimäres Fab, Fab' oder F(ab')₂ Immunoglobulin-Fragment ist, das umfaßt:
(a) Antigen-Bindungsregionen murinen Ursprungs, die spezifisch für das von dem murinen monoklonalen Antikörper 17-1A definierte Antigen sind; und
(b) eine menschliche konstante Region, wobei das chimäre Immunoglobulin eine Bindungsspezifität für besagtes Antigen beibehält.

5. Ein Verfahren zur Herstellung eines Reagenz zur Tumor-Szintigraphie, das den Schritt der Markierung des in Anspruch 4 definierten chimären Immunoglobulin-Fragments mit einem Radioisotop umfaßt, das aus der Gruppe gewählt ist, die aus ¹³¹Jod, ¹²⁵Jod, ^{99m}Technetium und ¹¹¹Indium besteht.

6. Das Verfahren nach Anspruch 4, worin das chimäre Immunoglobulin-Fragment außerdem umfaßt:
(c) ein metallbindendes Protein (z. B. Metallothionein) oder eine mit der konstanten Region verknüpfte Protein-Domäne.

7. Ein Verfahren zur Herstellung eines fusionierten Gens, das ein chimäres Murin/Mensch-Immunoglobulin codiert, das spezifisch für das von dem murinen monoklonalen Antikörper 17-1A definierte Antigen ist, wobei das Verfahren den Schritt der Verknüpfung:
a) einer ersten DNS-Sequenz, die mindestens den funktionalen Teil einer variablen Region einer murinen Immunoglobulin-Kette codiert, mit:
b) einer zweiten DNS-Sequenz, die mindestens einen Teil einer konstanten Region eines menschlichen Immunoglobulins codiert, umfaßt.

8. Das Verfahren nach Anspruch 7, worin das fusionierte Gen außerdem umfaßt:
c) eine dritte DNS-Sequenz, die ein Nicht-Immunoglobulin-Protein oder Peptid (besagtes Protein oder Peptid ist beispielsweise ein therapeutisches Mittel gegen Krebs, ein cytotoxisches Mittel, ein metallbindendes Mittel oder eine funktionale Domäne davon) codiert, wobei die Sequenz mit dem 3' Ende der zweiten DNS-Sequenz verknüpft ist.

9. Ein wie in Anspruch 1 definiertes chimäres Immunoglobulin oder Immunoglobulin-Fragment oder ein wie in Anspruch 4 definiertes Fragment zur Verwendung in Therapie oder Diagnose.

10. Ein wie in Anspruch 1 definiertes chimäres Immunoglobulin zur Verwendung in der Immuntherapie eines gastrointestinalen Tumors.

11. Ein chimäres Immunoglobulin zur Tumor-Immuntherapie, das umfaßt:
a) mindestens eine chimäre schwere Kette, die eine von der schweren Kette eines murinen Antikörpers stammende Antigen-Bindungsregion aufweist, die spezifisch für das von dem murinen monoklonalen Antikörper 17-1A definierten Antigen ist, verknüpft mit einer konstanten Region einer menschlichen schweren Kette und assoziiert damit:
b) mindestens eine chimäre leichte Kette, die eine von einer leichten Kette eines murinen Antikörpers stammende Antigen-Bindungsregion aufweist, die mit einer konstanten Region der menschlichen leichten Kette verknüpft ist, und
c) ein Antitumor-Therapeutikum oder ein cytotoxisches Mittel, das mit mindestens einer der konstanten Regionen verknüpft ist.

12. Verwendung eines wie in Anspruch 1 definierten chimären Immunoglobulins oder Immunoglobulin-Fragments, eines wie in Anspruch 4 definierten Fragments oder eines wie in Anspruch 7 definierten fusionierten Gens zur Herstellung eines Medikaments oder diagnostischen Mittels, z. B. zur Tumor-Immuntherapie oder Tumor-Diagnose.

## Revendications (Revendications pour l'(les) Etat(s) contractant(s) suivant(s): BE, CH, DE, FR, GB, IT, LI, LU, NL, SE)

1. Immunoglobuline chimérique murine/humaine ou fragment chimérique de celle-ci, comprenant une région de liaison dérivée d'un anticorps d'origine murine (par exemple de souris) spécifique de l'antigène défini par l'anticorps monoclonal murin 17-1A liée à au moins une partie d'une région constante d'origine humaine; l'immunoglobuline chimérique ou le fragment d'immunoglobuline conservant la spécificité de liaison audit antigène.

2. Immunoglobuline chimérique comprenant:
(a) au moins une chaîne lourde chimérique comprenant une région de liaison à l'antigène dérivée de la chaîne lourde d'un anticorps murin spécifique de l'antigène défini par l'anticorps monoclonal murin 17-1A liée à une région constante d'une chaîne lourde d'origine humaine (par exemple de type gamma) en association avec:
(b) au moins une chaîne légère chimérique comprenant une région de liaison à l'antigène dérivée d'une chaîne légère de l'anticorps murin liée à une région constante d'une chaîne légère d'origine humaine; l'immunoglobuline chimérique conservant la spécificité de liaison à l'antigène défini par l'anticorps monoclonal murin 17-1A.

3. Immunoglobuline chimérique comprenant:
(a) au moins une chaîne lourde chimérique comprenant une région de liaison à l'antigène dérivée de la chaîne lourde d'un anticorps murin spécifique de l'antigène défini par l'anticorps monoclonal murin 17-1A liée à une région constante d'une chaîne lourde d'origine humaine (par exemple de type gamma) en association avec:
(b) au moins une chaîne légère chimérique comprenant une région de liaison à l'antigène dérivée d'une chaîne légère de l'anticorps murin liée à une région constante d'une chaîne légère d'origine humaine; et
(c) une protéine ou un peptide autre qu'une immunoglobuline (ledit peptide ou protéine étant par exemple un agent thérapeutique anticancéreux, un agent cytotoxique, un agent de liaison à un métal ou un domaine fonctionnel de ceux-ci) lié au moins à l'une des régions constantes d'origine humaine; l'immunoglobuline chimérique conservant la spécificité de liaison à l'antigène défini par l'anticorps monoclonal murin 17-1A.

4. Fragment chimérique Fab, Fab' ou F(ab')₂ d'immunoglobuline, comprenant:
(a) des régions de liaison à l'antigène d'origine murine spécifiques de l'antigène défini par l'anticorps monoclonal murin 17-1A; et
(b) une région constante d'origine humaine; l'immunoglobuline chimérique conservant la spécificité de liaison audit antigène.

5. Réactif destiné à la scintigraphie tumorale comprenant le fragment d'immunoglobuline chimérique selon la revendication 4 marqué avec un isotope radioactif choisi au sein d'un groupe constitué par l'¹³¹iode, l'¹²⁵iode, le ^{99m}Technetium et l'¹¹¹Indium.

6. Fragment d'immunoglobuline chimérique selon la revendication 4 comprenant en outre:
(c) une protéine ou un domaine protéique qui se lie à un métal (par exemple la métallothionéine) lié à la région constante.

7. Gène fusionné codant pour une immunoglobuline chimérique murine/humaine spécifique de l'antigène défini par l'anticorps monoclonal murin 17-1A, comprenant :
(a) une première séquence d'ADN codant pour au moins la partie fonctionnelle d'une région variable d'une chaîne d'immunoglobuline murine, liée à
(b) une seconde séquence d'ADN codant pour au moins une partie d'une région constante d'une immunoglobuline humaine.

8. Gène fusionné selon la revendication 7 comprenant en outre:
c) une troisième séquence d'ADN codant pour une protéine ou un peptide autre qu'une immunoglobuline (ledit peptide ou protéine étant par exemple un agent thérapeutique anticancéreux, un agent cytotoxique, un agent de liaison à un métal ou un domaine fonctionnel de ceux-ci) liée à l'extrémité 3' de la seconde séquence d'ADN.

9. Immunoglobuline ou fragment d'immunoglobuline chimérique tel que défini à la revendication 1, ou fragment tel que défini à la revendication 4, destiné à être utilisé à des fins thérapeutiques ou diagnostiques.

10. Immunoglobuline chimérique telle que définie à la revendication 1 destinée à être utilisée dans l'immunothérapie d'une tumeur gastrointestinale.

11. Immunoglobuline chimérique destinée à être utilisée dans l'immunothérapie tumorale comprenant:
(a) au moins une chaîne lourde chimérique comprenant une région de liaison à l'antigène dérivée de la chaîne lourde d'un anticorps murin spécifique de l'antigène défini par l'anticorps monoclonal murin 17-1A liée à une région constante d'une chaîne lourde d'origine humaine en association avec:
(b) au moins une chaîne légère chimérique comprenant une région de liaison à l'antigène dérivée d'une chaîne légère d'un anticorps murin liée à une région constante d'une chaîne légère d'origine humaine; et
(c) un agent thérapeutique antitumoral ou cytotoxique lié au moins à l'une des régions constantes.

12. Utilisation d'une immunoglobuline ou fragment d'immunoglobuline chimérique tel que défini à la revendication 1, d'un fragment tel que défini à la revendication 4, ou d'un gène fusionné tel que défini à la revendication 7, dans la fabrication d'un médicament ou d'un agent de diagnostic, par exemple destiné à être utilisé dans l'immunothérapie ou le diagnostic des tumeurs.

## Revendications (Revendications pour l'(les) Etat(s) contractant(s) suivant(s): AT, ES, GR)

1. Procédé de préparation d'une immunoglobuline chimérique murine/humaine ou d'un fragment chimérique de celle-ci, comprenant une région de liaison à l'antigène issue d'un anticorps murin (par exemple de souris) spécifique de l'antigène défini par l'anticorps monoclonal murin 17-1A liée à au moins une partie d'une région constante d'origine humaine; l'immunoglobuline chimérique ou le fragment d'immunoglobuline conservant la spécificité de liaison audit antigène; ledit procédé comprenant les étapes de préparation, pour chacun des composants de la chaîne légère et de la chaîne lourde de l'immunoglobuline chimérique, d'un gène fusionné comprenant un premier fragment d'ADN qui code pour au moins la partie fonctionnelle de la région variable d'origine murine liée à un second segment d'ADN codant pour au moins une partie d'une région constante humaine.

2. Procédé de préparation d'une immunoglobuline chimérique comprenant:
(a) au moins une chaîne lourde chimérique comprenant une région de liaison à l'antigène dérivée de la chaîne lourde d'un anticorps murin spécifique de l'antigène défini par l'anticorps monoclonal murin 17-1A liée à une région constante d'une chaîne lourde d'origine humaine (par exemple de type gamma) en association avec:
(b) au moins une chaîne légère chimérique comprenant une région de liaison à l'antigène dérivée d'une chaîne légère de l'anticorps murin liée à une région constante d'une chaîne légère d'origine humaine; l'immunoglobuline chimérique conservant la spécificité de liaison à l'antigène défini par l'anticorps monoclonal murin 17-1A; ledit procédé comprenant les étapes de préparation, pour chacun des composants de la chaîne légère et de la chaîne lourde de l'immunoglobuline chimérique, d'un gène fusionné comprenant un premier fragment d'ADN qui code pour au moins la partie fonctionnelle de la région variable d'origine murine liée à un second segment d'ADN codant pour au moins une partie de la région constante humaine.

3. Procédé de préparation d'une immunoglobuline chimérique comprenant:
(a) au moins une chaîne lourde chimérique comprenant une région de liaison à l'antigène dérivée de la chaîne lourde d'un anticorps murin spécifique de l'antigène défini par l'anticorps monoclonal murin 17-1A liée à une région constante d'une chaîne lourde d'origine humaine (par exemple de type gamma) en association avec:
(b) au moins une chaîne légère chimérique comprenant une région de liaison à l'antigène dérivée d'une chaîne légère de l'anticorps murin liée à une région constante d'une chaîne légère d'origine humaine; et
(c) une protéine ou un peptide autre qu'une immunoglobuline (ledit peptide ou protéine étant par exemple un agent thérapeutique anticancéreux, un agent cytotoxique, un agent de liaison à un métal ou un domaine fonctionnel de ceux-ci) lié au moins à l'une des régions constantes d'origine humaine; l'immunoglobuline chimérique conservant la spécificité de liaison à l'antigène défini par l'anticorps monoclonal murin 17-1A; ledit procédé comprenant les étapes de préparation, pour chacun des composants de la chaîne légère et de la chaîne lourde de l'immunoglobuline chimérique, d'un gène fusionné comprenant un premier fragment d'ADN qui code pour au moins la partie fonctionnelle de la région variable d'origine murine lié à un second segment d'ADN codant pour au moins une partie d'une région constante d'origine humaine.

4. Procédé de la revendication 1, dans lequel le fragment chimérique est un fragment chimérique Fab, Fab' ou F(ab')2 d'immunoglobuline, comprenant:
(a) des régions de liaison à l'antigène d'origine murine spécifiques de l'antigène défini par l'anticorps monoclonal murin 17-1A; et
(b) une région constante d'origine humaine; l'immunoglobuline chimérique conservant la spécificité de liaison audit antigène.

5. Procédé de préparation d'un réactif destiné à la scintigraphie tumorale comprenant l'étape consistant à marquer le fragment d'immunoglobuline chimérique défini à la revendication 4 avec un isotope radioactif choisi au sein d'un groupe constitué par l'¹³¹iode, l'¹²⁵iode, le ^{99m}Technetium et l'¹¹¹Indium.

6. Procédé selon la revendication 4 dans lequel le fragment d'immunoglobuline chimérique comprend en outre:
(c) une protéine ou un domaine protéique qui se lie à un métal (par exemple la métallothionéine) lié à la région constante.

7. Procédé de fabrication d'un gène fusionné codant pour une immunoglobuline chimérique murine/humaine spécifique de l'antigène défini par l'anticorps monoclonal murin 17-1A, comprenant les étapes consistant à lier:
(a) une première séquence d'ADN codant pour au moins la partie fonctionnelle d'une région variable d'une chaîne d'immunoglobuline murine , à
(b) une seconde séquence d'ADN codant pour au moins une partie d'une région constante d'une immunoglobuline humaine.

8. Procédé selon la revendication 7 dans lequel le gène fusionné comprend en outre:
c) une troisième séquence d'ADN codant pour une protéine ou un peptide autre qu'une immunoglobuline (ledit peptide ou protéine étant par exemple un agent thérapeutique anticancéreux, un agent cytotoxique, un agent de liaison à un métal ou un domaine fonctionnel de ceux-ci) liée à l'extrémité 3' de la seconde séquence d'ADN.

9. Immunoglobuline ou fragment d'immunoglobuline chimérique tel que défini à la revendication 1, ou fragment tel que défini à la revendication 4, destiné à être utilisé à des fins thérapeutiques ou diagnostiques.

10. Immunoglobuline chimérique telle que définie à la revendication 1 destinée à être utilisée dans l'immunothérapie d'une tumeur gastrointestinale.

11. Immunoglobuline chimérique destinée à être utilisée dans l'immunothérapie tumorale comprenant:
(a) au moins une chaîne lourde chimérique comprenant une région de liaison à l'antigène dérivée de la chaîne lourde d'un anticorps murin spécifique de l'antigène défini par l'anticorps monoclonal murin 17-1A liée à une région constante d'une chaîne lourde d'origine humaine en association avec:
(b) au moins une chaîne légère chimérique comprenant une région de liaison à l'antigène dérivée d'une chaîne légère d'un anticorps murin liée à une région constante d'une chaîne légère d'origine humaine; et
(c) un agent thérapeutique antitumoral ou cytotoxique lié au moins à l'une des régions constantes.

12. Utilisation d'une immunoglobuline ou fragment d'immunoglobuline chimérique tel que défini à la revendication 1, d'un fragment tel que défini à la revendication 4, ou d'un gène fusionné tel que défini à la revendication 7, dans la fabrication d'un médicament ou d'un agent de diagnostic, par exemple destiné à être utilisé dans l'immunothérapie ou le diagnostic des tumeurs.
